# EUROPEAN PATENT APPLICATION

(11) **EP 2 623 213 A1**
(43) Date of publication of application: **07.08.2013**
(21) Application number: 11829072.5
(22) Date of filing: 27.09.2011
(51) Int. Cl.: B05C 5/04, A61F 13/15, A61F 13/49, B05B 15/04, B05D 1/02

(54) **ADHESIVE COATING DEVICE ACCORDING TO ABSORBENT ARTICLE AND ADHESIVE COATING METHOD**

(30) Priority: 28.09.2010 JP 2010217612
(71) Applicant: Uni-Charm Corporation, Ehime 799-0111 (JP)
(72) Inventor: TSUKUDA, Atsushi, Kanonji-shi Kagawa 769-1602 (JP); HASHIMOTO, Hiromitsu, Kanonji-shi Kagawa 769-1602 (JP)
(74) Representative: Peter, Julian
(86) International application number: PCT/JP2011/071968
(87) International publication number: WO 2012/043519

(57) **Abstract**

A device that applies adhesive on one side of a continuous sheet that travels, by acting air discharged from an air discharge hole of a head on the adhesive discharged from an adhesive discharge hole of the head. The device includes a first air curtain forming member positioned at a location on a downstream side of the head in a direction of travel of the continuous sheet, to oppose the head with a space therebetween, and forms a first air curtain by emitting air toward the one side of the continuous sheet. The device includes a pair of second air curtain forming members positioned at locations at outer sides from head in a width direction of the continuous sheet, to respectively oppose the head with spaces therebetween, and forms second air curtains by emitting air toward the one side of the continuous sheet.

## Description

### Technical Field

The present invention relates to an adhesive application device and an adhesive application method, used when manufacturing absorbent articles such as disposable diapers and the like, and applies adhesives such as hot-melt adhesives to continuous sheets of nonwoven fabric, films and the like.

### Background Art

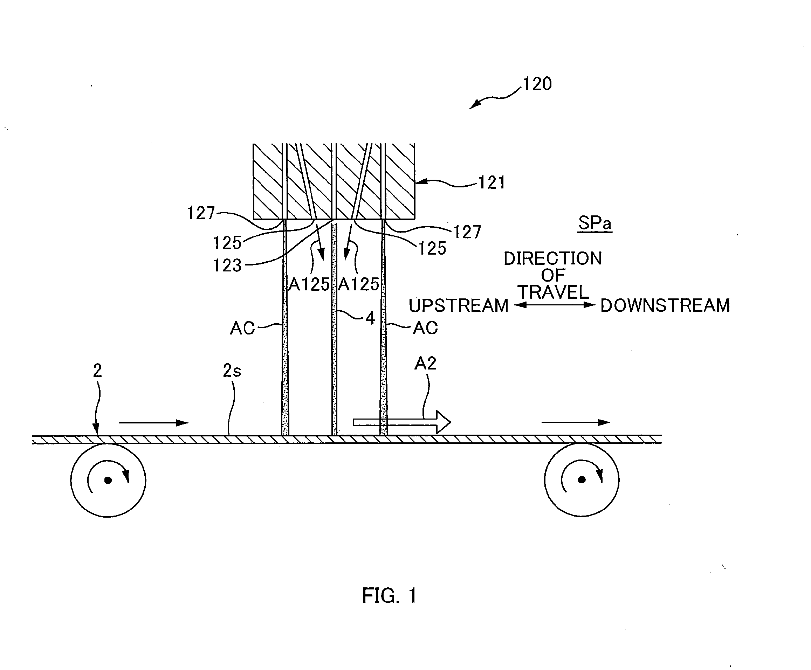

Conventionally, in a production line for disposable diapers and the like, as shown in a schematic side view in Fig. 1, the continuous sheet 2 made of such as nonwoven fabric or a film has been made to continuously travel in the direction of travel, and during this travel, hot-melt adhesives 4 have been applied to one side 2s of the continuous sheet 2.
This application is performed by a hot-melt adhesive application device 120 (hereafter referred to as HMA application device 120). The HMA application device 120 has, for example, a head 121 as the main body positioned at a predetermined position in the direction of travel of the continuous sheet 2, and said head 121 has an adhesive discharge hole 123 directed toward said one side 2s of the continuous sheet 2, and an air discharge hole 125 positioned adjacent the adhesive discharge hole 123. And the adhesive 4 is blown to be applied to the one side 2s of the continuous sheet 2 in a predetermined application pattern, by making air A125 discharged from the air discharge holes 125, 125 act on the adhesive 4 discharged from the adhesive discharge hole 123.
Here, PTL 1 discloses further in addition to the aforementioned adhesive discharge hole 123 and the air discharge hole 125, emitting holes 127, 127 that emit air for air curtains AC are provided to the head 121 and thereby foreign matters such as dust is prevented from sticking to the head 121.

### Citation List

### [Patent Literature]

[PTL 1] Japanese Patent Application Laid-open Publication No. 2002-102765

### Summary of Invention

### Technical Problem

However, PTL1 says nothing about the problem of the adhesive 4 scattering into the space SPa around the head 121. In other words, the problem of air A125 discharged from the air discharge holes 125, 125 of the head 121 reflected and the like by the continuous sheet 2 to scatter adhesives 4 into said surrounding space SPa, and the problem of airflow A2 generated by the travelling of the continuous sheet 2 spreading the scattering to the downstream side in the direction of travel is not mentioned at all. For such reason, measures for solving these problems have not been taken at all. As a result, there were risks of adhesives 4 scattering to spatter over the other devices located in said surrounding space SPa, and increasing the cleaning frequency to inhibit productivity.

The present invention has been made in view of conventional problems such as those mentioned above, and an object thereof is to restrain adhesives from scattering into the space around the head.

### Solution to Problem,

In order to solve the above-described problem, a principal aspect of the invention is, an adhesive application device that applies adhesive on one side of a continuous sheet that travels, by acting air discharged from an air discharge hole of a head on the adhesive discharged from an adhesive discharge hole of the head, the adhesive application device related to an absorbent article including a first air curtain forming member positioned at a location on a downstream side of the head in a direction of travel of the continuous sheet, to oppose the head with a space therebetween, and forms a first air curtain by emitting air toward the one side of the continuous sheet; and a pair of second air curtain forming members positioned at locations at outer sides from head in a width direction of the continuous sheet, to respectively oppose the head with spaces therebetween, and forms second air curtains by emitting air toward the one side of the continuous sheet.
And a further aspect of the invention is, a method of applying adhesive on one side of a continuous sheet that travels, by acting air discharged from an air discharge hole of a head on the adhesive discharged from an adhesive discharge hole of the head, the adhesive application method related to an absorbent article including forming a first air curtain by positioning a first air curtain forming member at a location on a downstream side of the head in a direction of travel of the continuous sheet, to oppose the head with a space therebetween, and emitting from the first air curtain forming member air toward the one side of the continuous sheet; and forming second air curtains by positioning second air curtain forming members at locations at outer sides from head in a width direction of the continuous sheet, to respectively oppose the head with spaces therebetween, and emitting from the second air curtain forming members air toward the one side of the continuous sheet.

Features of the invention other than the above will become clear from the description of the present specification and the drawings attached.

### Advantageous Effects of Invention

According to the present invention, it is possible to restrain scattering of adhesives into the space around the head.

### Brief Description of Drawings

Fig. 1 is a schematic side view of a conventional HMA application device 120.
Fig. 2A is a schematic perspective view of the HMA application device 20 of the present embodiment seen from the head 21 side.
Fig. 2B is a schematic perspective view of the same HMA application device 20 seen from the continuous sheet 2 side.
Fig. 3A is a schematic vertical sectional view seen through the center of the HMA application device 20.
Fig. 3B is a diagram showing view B-B in Fig. 3A.
Fig. 3C is a diagram showing view C-C in Fig. 3A.
Fig. 4 is an explanatory view of the air curtain forming member 41a according to another embodiment.
Fig. 5 is an explanatory view of the air curtain forming member 41b according to another embodiment.

### Mode for Carrying Out the Invention

At least the following matters will be made clear from the description of the present specification with reference to the accompanying drawings.

First, the adhesive application device related to an absorbent article according to the present invention is a device that applies adhesive on one side of a continuous sheet that travels, by acting air discharged from an air discharge hole of a head on the adhesive discharged from an adhesive discharge hole of the head, the adhesive application device related to an absorbent article including a first air curtain forming member positioned at a location on a downstream side of the head in a direction of travel of the continuous sheet, to oppose the head with a space therebetween, and forms a first air curtain by emitting air toward the one side of the continuous sheet, and a pair of second air curtain forming members positioned at locations at outer sides from head in a width direction of the continuous sheet, to respectively oppose the head with spaces therebetween, and forms second air curtains by emitting air toward the one side of the continuous sheet.
According to such an adhesive application device related to an absorbent article, a space is provided between the head and the first air curtain forming member, and spaces are also provided between the head and the second air curtain forming members. And as a result of air in these spaces respectively accompanying the air curtains, accompanying air streams toward the continuous sheet flow through these spaces. Therefore, besides the air curtains, these accompanying air streams restrain scattering of adhesives into the space surrounding the head. As a result, antiscattering effect of the adhesives can be expanded.

Further, owing to the airstream generated by the travelling of the continuous sheet, adhesive is likely to scatter downstream the direction of travel than upstream thereof. However according to the aforementioned configuration, since at a location downstream the direction of travel, air curtains are formed to surround such location by the first air curtain forming member and a pair of second air curtain members, such scattering of adhesives can be effectively restrained.

It is preferable that in the adhesive application device related to an absorbent article the first air curtain forming member and the pair of second air curtain forming members, by being continuously provided to one another, are positioned to surround the head from at least three sides being a downstream side in the direction of travel and both sides in the width direction, the first emitting hole of a slit shape that emits the air relating to the first air curtain is provided to a face, of the first air curtain forming member, that opposes the one side of the continuous sheet, the second emitting holes of slit shapes that emit the air relating to the second air curtains are provided to each faces, of the pair of second air curtain forming members, that oppose the one side of the continuous sheet, and the first emitting hole is connected to the second emitting holes.
According to such an adhesive application device related to an absorbent article, the first air curtain of the first air curtain forming member, and the second air curtain of the second air curtain forming member are continuously formed so that discontinuity such as a gap and the like is not created therebetween. Therefore, the head can be certainly surrounded by air curtains from three directions, being the downstream side in the direction of travel and the two sides in said width direction and thereby, scattering of adhesives into the surrounding space can be effectively restrained.

It is preferable that in the adhesive application device related to an absorbent article the first air curtain forming member has a first wall that opposes the head, the pair of second air curtain forming members respectively have a second wall each opposing the head, and the first wall and the second walls are each positioned to extend to a location distant from the one face than a location of the adhesive discharge hole in relation to a direction normal to the one side of the continuous sheet.
According to such an adhesive application device related to absorbent articles, said wall is provided to extend up to a location distant from the continuous sheet than the adhesive discharge hole. Thereby, accompanying airflows that may be generated in the space between the air curtain forming members and the head, can be guided from a location distant from the adhesive discharge hole. In this way, said accompanying airflows can be certainly mode to act on the adhesives immediately after being discharged from the adhesive discharge holes. And as a result, the effect of restraining scattering of adhesives into the surrounding space can be improved.

It is preferable that in the adhesive application device related to an absorbent article the first emitting hole of the first air curtain forming member and the second emitting holes of the pair of second air curtain forming members are each positioned at a location distant from the one face than a location of the adhesive discharge hole of the head in relation to a direction normal to the one side of the continuous sheet.
According to such an adhesive application device related to an absorbent article, the scattered adhesives can be effectively restrained from sticking to the first emitting hole and the second emitting holes, so the first air curtain and the second air curtains can be certainly formed.

It is preferable that in the adhesive application device related to an absorbent article a third air curtain forming member is positioned at a location on an upstream side of the head in a direction of travel of the continuous sheet, to oppose the head with a space therebetween, and forms a third air curtain by emitting air toward the one side of the continuous sheet.
According to such an adhesive application device related to an absorbent article, scattering of adhesives to the upstream side can be restrained. Further, said space between the head and the third air curtain forming member has formed thereto an accompanying airflow that accompanies the third air curtain thus scattering of adhesives to the upstream side in the direction of travel can be further effectively restrained.

It is preferable that in the adhesive application device related to an absorbent article the pair of second air curtain forming members, by being continuously provided to the first air curtain forming member and the third air curtain forming member, are positioned to surround the head from at least four sides being a downstream side and upstream side in the direction of travel and both sides in the width direction, the first emitting hole of a slit shape that emits the air related to the first air curtain is provided to a face, of the first air curtain forming member, that opposes the one side of the continuous sheet, the second emitting holes of slit shapes that emit the air relating to the second air curtains are provided to each faces, of the pair of second air curtain forming members, that oppose the one side of the continuous sheet, the third emitting hole of a slit shape that emits the air relating to the third air curtain is provided to a face, of the third air curtain forming member, that opposes the one side of the continuous sheet, and the second emitting holes are connected to the first emitting hole and the third emitting hole.
According to such an adhesive application device related to an absorbent article, the second air curtains of the pair of second air curtain forming members, and the first air curtain of the first air curtain forming member and the third air curtain of the third air curtain forming member, are continuously formed to each other so that discontinuity such as a gap and the like is not created therebetween. Therefore, the head can be certainly enclosed by aircurtains from four sides, being both upstream and downstream sides of the direction of travel and the two sides in said width direction and thereby, scattering of adhesives into the surrounding space can be effectively restrained.

Further, the adhesive application method related to an absorbent article according to the present invention is a method of applying adhesive on one side of a continuous sheet that travels, by acting air discharged from an air discharge hole of a head on the adhesive discharged from an adhesive discharge hole of the head, the adhesive application method related to an absorbent article including forming a first air curtain by positioning a first air curtain forming member at a location on a downstream side of the head in a direction of travel of the continuous sheet, to oppose the head with a space therebetween, and emitting from the first air curtain forming member air toward the one side of the continuous sheet, and forming second air curtains by positioning second air curtain forming members at locations at outer sides from head in a width direction of the continuous sheet, to respectively oppose the head with spaces therebetween, and emitting from the second air curtain forming members air toward the one side of the continuous sheet.
According to such adhesive application method related to absorbent articles, a space is provided between the head and the first air curtain forming member, and spaces are also provided between the head and the second air curtain forming members. And as a result of air in these spaces respectively accompanying the air curtains, accompanying air streams toward the continuous sheet flow through these spaces. Therefore, besides the air curtains, these accompanying air streams restrain scattering of adhesives into the space surrounding the head. As a result, antiscattering effect on the adhesives can be expanded.

Further, owing to the airstream generated by the travelling of the continuous sheet, adhesive is likely to scatter downstream the direction of travel than upstream thereof. However according to the aforementioned configuration, since at a location downstream the direction of travel, air curtains are formed to surround such location by the first air curtain forming member and a pair of second air curtain members, such scattering of adhesives can be effectively restrained.

### === The Present Embodiment ===

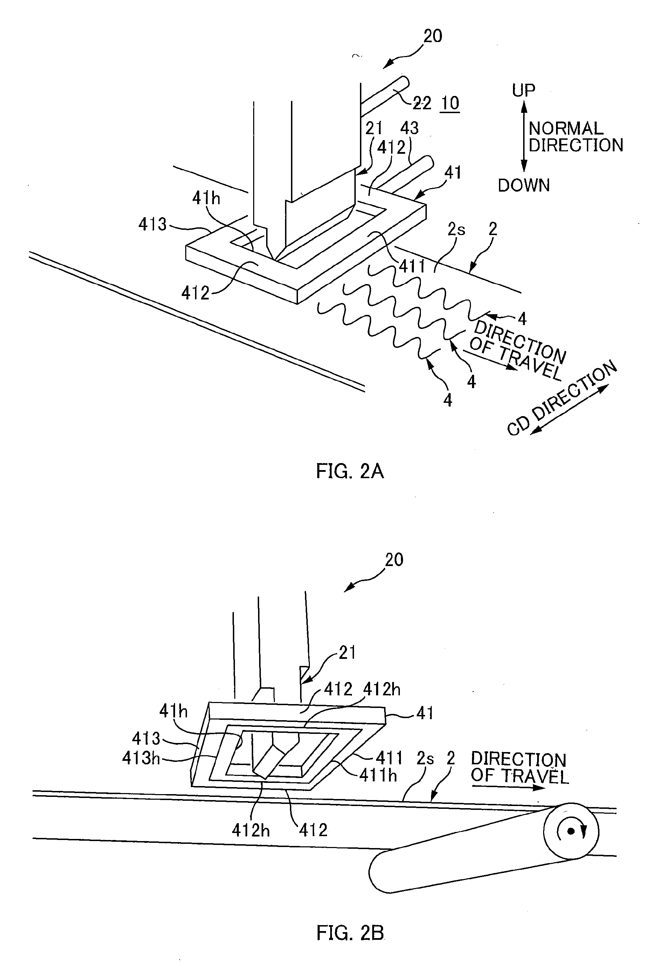

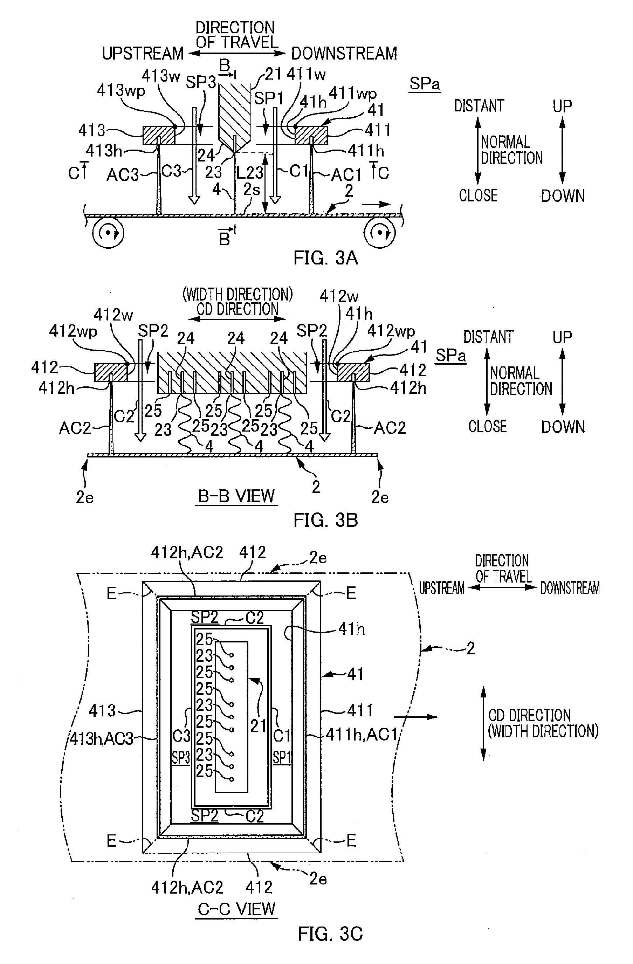

Figs. 2A to 3C are explanatory diagrams of the adhesive application device according to the present embodiment. Fig. 2A is a schematic perspective view of the same application device 20 seen from the head 21 side, and Fig. 2B is a schematic perspective view of the same application device 20 seen from the continuous sheet 2 side. Further, Fig. 3A is a schematic vertical sectional view seen through the center of the same application device 20, and Figs. 3B and 3C are B-B and C-C views, respectively in Fig. 3A.

This adhesive application device 20 is used in manufacturing lines for absorbent articles such as disposable diapers and sanitary napkins. In other words, as shown in Fig. 2A, the same application device 20 is for applying adhesive 4 in a predetermined pattern, to one side 2s of the continuous sheet 2 that travels in a predetermined direction of travel. And at a location to the downstream side, than this application device 20, in the direction of travel, separate continuous sheets or cut sheets (not shown) and the like as separate parts are appropriately overlapped and joined into one piece at the portion where the same adhesive 4 is applied to be successively transported to the following process as a partially-finished product of an absorbent article.

As the continuous sheet 2, for example, there are nonwoven fabric, fabric, film and the like, to name a few. And as an example of material therefor, there is thermoplastic resin however, the material is not limited to such and pulp fiber and the like may be used. Further, regarding the air permeability in the thickness direction, since air can easily escape in the thickness direction from sheets with high permeability thus sheets with high permeability is preferred from the viewpoint of restraining the scattering of adhesives 4 however, non-permeable sheets may be used.

As adhesive 4, hot-melt adhesive can be given as an example however, it is not limited to such. That is, adhesives besides hot-melt adhesives may be used as long as the adhesive 4 has appropriate thermoplasticity and can be applied in a fluid state by being melted by heat. Note that, in the following, description will be given in the case hot-melt adhesive is used, and the same adhesive 4 is also referred to as simply adhesive 4, and the adhesive application device 20 is also referred to as HMA application device 20.

Further in the following description, the direction of travel of the continuous sheet 2 that travels along the manufacturing line is also referred to as the MD direction, and the width direction of the same continuous sheet 2 is also referred to as the CD direction. Note that in Fig. 3A, the MD direction is an arbitrary direction parallel with the plane of the paper of Fig. 3A, and the same MD direction also includes the up-down direction. Further, the CD direction is the direction that penetrates the plane of the paper of Fig. 3A, and the same direction is also referred to as the right-left direction.

The HMA application device 20 is provided to oppose one side 2s of the continuous sheet 2, and includes a head 21 that discharges adhesive 4 from the adhesive discharge hole 23 toward the same one side 2s, and an air curtain forming member 41 that forms the air curtains AC1, AC2, AC2, AC3 around the head 21 to restrain scattering of the adhesive 4 discharged from the head 21 into the surrounding space SPa.

As shown in Fig. 2A, the head 21 is fixed in a manner prevented from moving at a predetermined position in the direction of travel of the continuous sheet 2. For example, the head 21 is fixed and supported, in a manner prevented from moving, to an appropriate support member 10 such as a mirror plate via an appropriate stay member 22. Further as shown in Fig. 3A, the adhesion discharge hole 23 of the head 21 is position to oppose the one side 2s of the continuous sheet 2. And a flow channel 24 that is in fluid communication with the adhesive discharge hole 23 is formed inside the head 21, and adhesive 4 is forced into the flow channel 24 by a pump, not shown, provided outside, and thereby adhesive 4 is discharged from the adhesive discharge hole 23. Note that, a valve that opens/closes the flow channel 24 may be provided to control starting and stopping of discharge.

Further, as shown in Figs. 3B and 3C, a pair of right and left air discharge holes 25, 25 is positioned at both side locations in the CD direction of the adhesive discharge hole 23 of the head 21. And air discharged from these air discharge holes 25, 25 act on the adhesive 4 discharged from the adhesive discharge hole 23 and thereby, adhesive 4 is applied to the continuous sheet 2 in a predetermined application pattern.

For example, air is alternately discharged at a predetermined cycle from the pair of air discharge holes 25, 25. Then as a result of the adhesive 4 being attracted to the direction of air discharge by a so-called joining effect, adhesive 4 is blown toward the continuous sheet 2 and applied thereto in an S-shape pattern of a substantially sinusoidal waveform as shown in Fig. 3B.

Note that in the example shown, three adhesive discharge holes 23 provided with such air discharge holes 25, 25 are aligned in the CD direction and thereby, three lines of adhesives 4 are applied in a substantially sinusoidal waveform to the continuous sheet 2 however, the number of lines is not limited to three. Additionally, the pattern in which adhesive 4 is applied is not limited to an S-shape pattern of a substantially sinusoidal waveform and can be applied in various patterns. For example, a spiral pattern may be adopted. In such case, said spiral pattern can be realized by the plurality of air discharge holes 25, 25 ... being positioned at a predetermined pitch along a circle with its center at the adhesive discharge hole 23, and acting air discharged from the air discharge holes 25, ... with an angle toward adhesive 4 discharged from the adhesive discharge hole 23.

As shown in Fig. 3C, the air curtain forming member 41 that has its main body a member in an approximately frame form, in a planar view, made by an approximately rectangular shaped opening 41h penetrating the inner side of an approximately rectangular plate form member. And the head 21 is positioned at approximately the center of this opening 41h. Thereby, the air curtain forming member 41 is positioned to surround the head 21 from four sides being two sides in the CD direction and two sides in the direction of travel (MD direction).

As shown in Figs. 3A to 3C, this air curtain forming member 41 can be divided into four parts. In other words, four members being, the first air curtain forming member 411 positioned at a location downstream of the head 21 in the direction of travel and forming the first air curtain AC1 along the CD direction at said location, right-left pair of second air curtain forming members 412, 412 positioned at locations at the outer sides in the CD direction from the head 21 and forming the second air curtains AC2 along the direction of travel at said locations, and the third air curtain forming member 413 positioned at a location upstream of the head 21 in the direction of travel and forming the third air curtain AC3 along the CD direction at said location. And the right-left pair of second air curtain forming members 412, 412 are continuous by being respectively connected to the first air curtain forming member 411 and the third air curtain forming member 413 at each end portions, and thereby the air curtain forming member 41 as whole is in the above-mentioned approximately frame form.

As shown in Fig. 3C, the air curtain forming members 411, 412, 412, 413 respectively include slit shaped emitting holes 411h, 412h, 412h, 413h at the face opposing the continuous sheet 2, and form the aforementioned air curtains AC1, AC2, AC2, AC3 by emitting air toward the continuous sheet 2 from the emitting holes 411h, 412h, 412h, 413h, respectively. Further, the emitting holes 412h, 412h of the right-left pair of second air curtain forming members 412, 412 are respectively connected to the emitting hole 411h of the first air curtain forming member 411 and the emitting hole 413h of the third air curtain forming member 413 at each end portions.

In this way, the right-left pair of second air curtains AC2, AC2 are also respectively connected to the first air curtain AC1 and the third air curtain AC3 at each end portions, and these connected four air curtains AC1, AC2, AC2, AC3 as a whole form a single rectangular tube air curtain (hereafter called air curtain tube). And with this air curtain tube, the head 21 can be surrounded from four sides being the two sides in the CD direction and the two sides in the direction of travel without discontinuity such as a gap and the like along the whole circumference thereof. And as a result, adhesive 4 applied from the head 21 toward the one side 2s of the continuous sheet 2 can be effectively restrained from scattering into the surrounding space SPa.

By the way, as can be seen with reference to Figs. 3A to 3C, the aforementioned four air curtain forming members 411, 412, 412, 413 are respectively positioned to oppose the head 21 in a state with spaces SP1, SP2, SP2, SP3 between the head 21, in the present embodiment. To be specific, the head 21 and the four air curtain forming members 411, 412, 412, 413 have therebetween spaces SP1, SP2, SP2, SP3 respectively formed along the direction normal to the one side 2s of the continuous sheet 2, and these spaces SP1, SP2, SP2, SP3 are respectively in fluid communication with the surrounding space SPa.

Therefore, the spaces SP1, SP2, SP2, SP3 has formed thereto airflows C1, C2, C2, C3 (hereafter also referred to as accompanying airflows C1, C2, C2, C3) along the aforementioned normal direction accompanying the air of the air curtains AC1, AC2, AC2, AC3 flowing proximate the spaces SP1, SP2, SP2, SP3. In other words, the space SP1 between the first air curtain forming member 411 and the head 21 has formed an accompanying airflow C1 that flows in the direction same as the flow direction of the first air curtain AC1, the space SP2 between the second air curtain forming member 412 and the head 21 has formed an accompanying airflow C2 that flows in the direction same as the flow direction of the second air curtain AC2, and the space SP3 between the third air curtain forming member 413 and the head 21 has formed an accompanying airflow C3 that flows in the direction same as the flow direction of the third air curtain AC3. And theses accompanying airflows C1, C2, C2, C3 assists in the restraining of adhesive 4 from scattering by the first through third air curtains AC1, AC2, AC2, AC3 to further effectively restrain the adhesive 4 from scattering into the surrounding space SPa.

Further as shown in Fig. 3C, the aforementioned spaces SP1, SP2, SP2, SP3 are interconnected at the end portions and as a result, form a single annular space surrounding the head 21. Thereby, the aforementioned accompanying airflows C1, C2, C2, C3 are also interconnected at the end portions so that these accompanying airflows C1, C2, C2, C3 are also integrated into as a single annular flow. In other words, these accompanying airflows C1, C2, C2, C3 take on the semblance of an annular flow aligned at the inner circumferential side of the air curtain tube. And since the head 21 is surrounded also by said annular flow, from four sides being the two sides in the direction of travel and the two sides in C the D direction, without discontinuity such as a gap and the like around its whole circumference, scattering of adhesive 4 to the surrounding space SPa can be much more effectively restrained.

Note that, the head 21 and the air curtain forming member 41 are avoided from being physically connected so to allow to form a successive annular space where said spaces SP1, SP2, SP2, SP3 are continuous. In other words, as shown in Fig. 2A, in the present embodiment, the aforementioned head 21 is not used as a support member for positioning the air curtain forming member 41 around the head 21 while supporting the same member 41, and the air curtain forming member 41 is fixedly supported by the support member 10 of the aforementioned mirror plate and the like via a dedicated stay member 43.

By the way, it is preferable that the positional relation of the adhesive discharge hole 23 of the head 21 and the air curtain forming member 41 are set in the following manner.

Firstly, it is preferable that the walls 411w, 412w, 412w, 413w that oppose the head 21 among the plurality of walls provided to the air curtain forming members 411, 412, 412, 413 are extended up to a distant location from the one side 2s of the continuous sheet 2 than the location of the adhesive discharge hole 23 as to the direction normal to the one side 2s of the continuous sheet 2. To be specific, it preferable that with regard to the walls 411w, 412w, 412w, 413w, the distance in the normal direction from the locations 411wp, 412wp, 412wp, 413wp farthest from the aforementioned one side 2s to the same one side 2s is greater than the distance L23 in the aforementioned normal direction from the one side 2s of the continuous sheet 2 to the adhesive discharge hole 23.

And when arranged in such way, the accompanying airflows C1, C2, C2, C3 that may be generated in the spaces SP1, SP2, SP2, SP3 can be guided from a location (from the upper side in Figs. 3A and 3B) farther than the adhesive discharge hole 23. And thereby, these accompanying airflows C1, C2, C2, C3 can be certainly acted on the adhesive 4 immediately after discharge from the adhesive discharge hole 23. And as a result, the effect of restraining the adhesive 4 from scattering into the surrounding space SPa can be improved.

Further, more preferably, the locations at which the emitting holes 411h, 412h, 412h, 413h of the air curtain forming members 411, 412, 412, 413 are positioned, are set farther in said normal direction from the one side 2s of the continuous sheet 2 than the location at which the adhesive discharge hole 23 of the respective heads 21. To be specific, it is preferable that the distance in the aforementioned normal direction from the aforementioned one side 2s to the emitting hoes 411h, 412h, 412h, 413h is greater than distance L23 in the same normal direction from the aforementioned one side 2s to the discharge hole 23.

And when arranged in such way, sticking of the scattering adhesive 4 to the emitting holes 411h, 412h, 412h, 413h or their surrounding areas can be effectively restrained and the accuracy in forming the air curtains AC1, AC2, AC2, AC3 can be improved through restraining clogging of the emitting holes 411h, 412h, 412h, 413h.

Note that, the direction of adhesive 4 discharged from the adhesive discharge hole 23 of the head 21 and the direction of air discharged from the air discharge hole 25, and the direction of air emitted from the emitting holes 411h, 412h, 412h, 413h, are all basically specified to be in directions in parallel with the normal direction of the one side 2s of the continuous sheet 2. However, the directions are not limited to such and may be, for example, inclined from said normal direction at an angle within the range of ±10 degrees (more preferably within the range of ±5 degrees). And when within this range, the scattering prevention effect of the adhesive 4 is can be effectively exercised however, the same effect will deteriorate when the direction is beyond this range.

Further, the distance from the adhesive discharge hole 23 of the head 21 to the one side 2s of the continuous sheet 2 is selected from the range of, for example, 20mm to 50mm. The adhesive 4 application width will be narrow when the distance is less than 20mm and the scattering of the adhesive 4 will increase to contribute to spattering the surroundings as well as cooling the adhesive 4 leading to deterioration of adhesive strength when the distance is greater than 50mm. Whereas, the distance from the air discharge hole 25 of the head 21 to the one side 2s of the continuous sheet 2 is selected from the range of, for example, 20mm to 50mm. This is because the discharged adhesive 4 is likely to stick onto the head 21, which is not desirable, unless the air discharge hole 25 is basically at the same position as the adhesive discharge hole 23 in relation to the position in said normal direction. Additionally, the distance between the emitting holes 411h, 412h, 412h, 413h of the air curtain forming members 411, 412, 412, 413 and the one side 2s of the continuous sheet 2 is selected from the range of, for example, 25mm to 55mm. And when set within this range, the emitting holes 411h, 412h, 412h, 413h of the air curtains AC1, AC2, AC2, AC3 can be positioned at a location distant from the continuous sheet 2 than the adhesive discharge hole 23. As a result, sticking of the adhesive 4 to the same emitting holes 411h, 412h, 412h, 413h can be prevented allowing to stably create the air curtains AC1, AC2, AC2, AC3.

Further, there may be a case where the third air curtain forming member 413 is omitted. The reason why this is possible is that an airflow is generated along the direction of travel (for example, refer to airflow A2 in Fig. 1) proximate the one side 2s of the continuous sheet 2, by the travelling of continuous sheet 2. And for this reason, scattering of adhesive 4 toward upstream the direction of travel will be relatively minor. Conversely, the amount of scattering of adhesive 4 toward downstream the direction of travel is likely to be excessively large due to the airflow in the same direction of travel therefore the first air curtain forming member 411 positioned at a location at the downstream side in the direction of travel than the head 21, becomes an essential member, and the pair of second air curtain forming members 412, 412 that restrain the scattering at the two sides in the CD direction also become essential members.

Among such methods of omitting the third air curtain forming member 413, there are for example, the following two methods. As the first method, an approximately frame form member 41 (refer to Fig. 3C) having the same structure as that mentioned above is used for the main body of the air curtain forming member 41 and only the first emitting hole 411h and the pair of second emitting holes 412h, 412h are formed, without the third emitting hole 413h being formed to said member 41, or if the third emitting hole 413h is already formed, the third emitting hole 413h is covered with a tape member and the like to prevent air from being emitted.

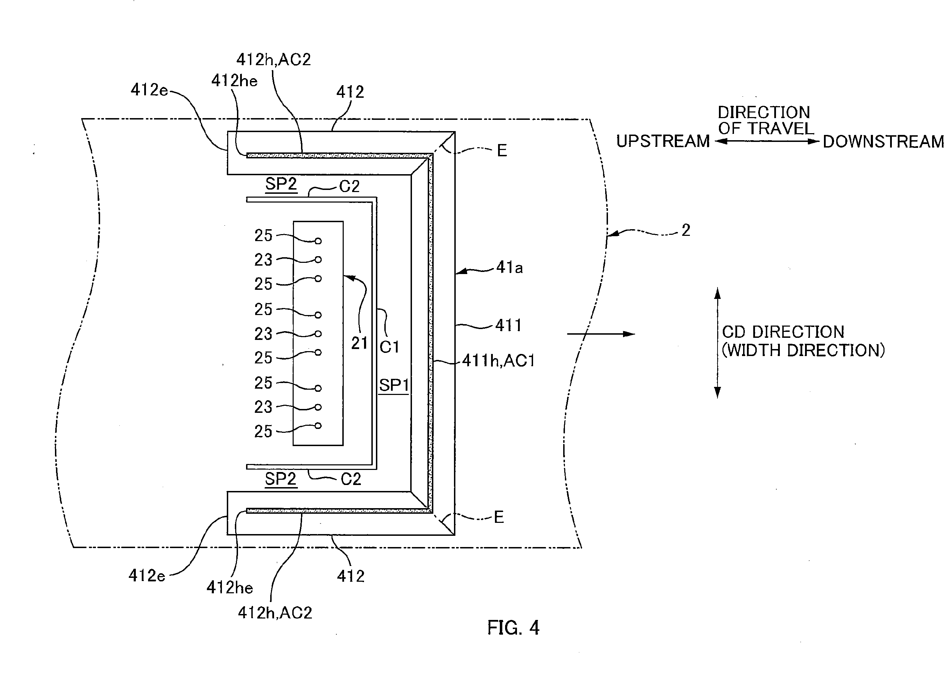

As the second method, the air curtain forming member 41a is made with an approximately U-shape member 41a as shown in Fig. 4 by such as removing the part corresponding to the third air curtain forming member of the air curtain forming member 41. Note that in such case, it is preferable that the second air curtain forming member 412 is positioned such that the end portion 412e of the second air curtain forming member 412, on the upstream side in the direction of travel, is position upstream of the head 21 in relation to the same direction of travel. In this way, the end portion 412he of the second emitting hole 412h can also be positioned on the upstream side of the head 21, and along with this, the range at which the accompanying flows C2, C2 are formed is expanded to the upstream side and as a result, scattering of adhesive 4 in the CD direction can be effectively restrained.

### === Other Embodiments ===

Hereinabove, explanation on the embodiments of the present invention have been given however, the present invention is not limited to such embodiments and modification such as those in the following can be made.

In the aforementioned embodiment, absorbent articles were described to be disposable diapers and sanitary napkins. However, the absorbent article is not limited to such as long as it absorbs liquid excretions such as urine, menses and the like, and can be, for example, pet waste pads that absorb liquid excretion of pets, and the like.

In the aforementioned embodiment, as shown in Fig. 3C, slit shaped emitting holes 411h, 412h, 412h, 413h were provided to the air curtain forming member 41 however, the shape of the emitting holes 411h, 412h, 412h, 413h is not limited to slits as long as an air curtain, in other words an air flow (stream of air) in a film state or a wall state can be formed. For example, a plurality of emitting holes of circular or polygonal shapes aligned along a predetermined direction can be used.

In the aforementioned embodiment, the direction of travel of the continuous sheet 2 was set approximately horizontal however, the direction of travel is not limited to such and can be in the vertical direction or in a direction inclining between the horizontal and vertical directions.

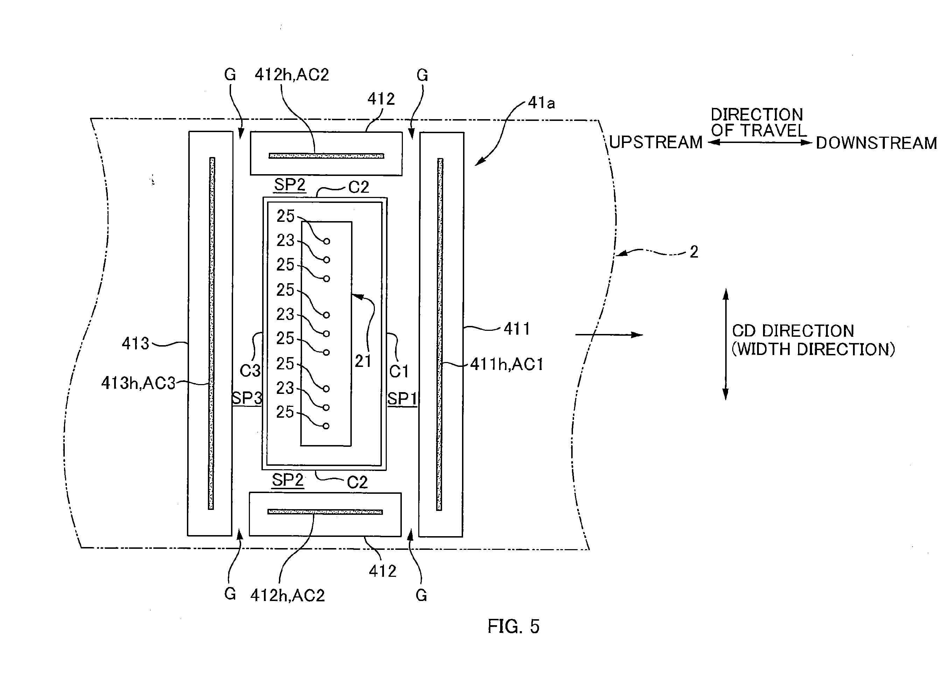

In the aforementioned embodiment, as shown in Fig. 3C, both of the second air curtain forming members 412, 412 were physically continuous with the first air curtain forming member 411 at the end portion E downstream in the direction of travel and physically continuous with the third air curtain forming member 413 at the end portion E upstream in the direction of travel as well. However, the relation is not limited to such. In other words, as the air curtain forming member 41b in Fig. 5, the air curtain forming member 41 can be divided at the end portion E or a portion proximate the end portion E with a gap G is formed between each other. Thereby, the first to the third air curtain forming members 411, 412, 412, 413 can be configured as an approximate rectangular parallelepiped rod member separate from each other.

However, in the case of this structure, the second emitting holes 412h, 412h cannot be connected to the first emitting hole 411h and the third emitting hole 413h, so that the air curtain tube being the tubular air curtain is not formed and the effect in restraining scattering of the adhesive 4 is regarded to decline and thus the configuration in the aforementioned embodiment is preferable from such viewpoint.

In the aforementioned embodiment, as shown in Figs. 3B and 3C, the location in the CD direction at which the second emitting hole 412h of the second air curtain forming member 412 is formed is located inside in the CD direction than the location of the edge 2e in the width direction (direction same as the CD direction) of the continuous sheet 2. And thereby, the second air curtain AC2 made by air emitted from the second emitting hole 412h is made to hit against the one side 2s of the continuous sheet 2 however, the position is not limited to such. In other words, there may be a case where the location at which the second emitting hole 412h is formed is located outside in the CD direction than said edge 2e of the continuous sheet 2. Note that in such case, it is a matter of course that the second air curtain AC2 would not hit against the one side 2s of the continuous sheet 2 however, the effect in restraining adhesive 4 from scattering outside in the CD direction is exercised since the second air curtain AC2 is formed.

### Reference Signs List

- 2: continuous sheet, 2e edge, 2s one side, 4 adhesive,
- 10: support member,
- 20: adhesive application device, 21 head, 22 stay member,
- 23: adhesive discharge hole, 24 flow channel, 25 air discharge hole,
- 41: air curtain forming member, 41a air curtain forming member,
- 41b: air curtain forming member, 41h opening, 43 stay member,
- 411: first air curtain forming member, 411h first emitting hole,
- 411w: wall (first wall), 411wp location,
- 412: second air curtain forming member, 412e end portion,
- 412h: second emitting hole, 412he edge portion,
- 412w: wall (second wall), 412wp location,
- 413: third air curtain forming member, 413h third emitting hole,
- 413w: wall, 413wp location,
- AC1: first air curtain, AC2 second air curtain,
- AC3: third air curtain,
- SP1: space, SP2 space, SP3 space, SPa surrounding space,
- C1: accompanying airflow, C2 accompanying airflow,
- C3: accompanying airflow,
- E: end portion, G gap

## Claims

1. An adhesive application device that applies adhesive on one side of a continuous sheet that travels, by acting air discharged from an air discharge hole of a head on the adhesive discharged from an adhesive discharge hole of the head, the adhesive application device related to an absorbent article comprising:
a first air curtain forming member positioned at a location on a downstream side of the head in a direction of travel of the continuous sheet, to oppose the head with a space therebetween, and forms a first air curtain by emitting air toward the one side of the continuous sheet; and
a pair of second air curtain forming members positioned at locations at outer sides from head in a width direction of the continuous sheet, to respectively oppose the head with spaces therebetween, and forms second air curtains by emitting air toward the one side of the continuous sheet.

2. An adhesive application device related to an absorbent article according to claim 1, wherein
the first air curtain forming member and the pair of second air curtain forming members, by being continuously provided to one another, are positioned to surround the head from at least three sides being a downstream side in the direction of travel and both sides in the width direction,
the first emitting hole of a slit shape that emits the air relating to the first air curtain is provided to a face, of the first air curtain forming member, that opposes the one side of the continuous sheet,
the second emitting holes of slit shapes that emit the air relating to the second air curtains are provided to each faces, of the pair of second air curtain forming members, that oppose the one side of the continuous sheet, and
the first emitting hole is connected to the second emitting holes.

3. An adhesive application device related to an absorbent article according to claim 2, wherein
the first air curtain forming member has a first wall that opposes the head,
the pair of second air curtain forming members respectively have a second wall each opposing the head, and
the first wall and the second walls are each positioned to extend to a location distant from the one face than a location of the adhesive discharge hole in relation to a direction normal to the one side of the continuous sheet.

4. An adhesive application device related to an absorbent article according to claim 3, wherein
the first emitting hole of the first air curtain forming member and the second emitting holes of the pair of second air curtain forming members are each positioned at a location distant from the one face than a location of the adhesive discharge hole of the head in relation to a direction normal to the one side of the continuous sheet.

5. An adhesive application device related to an absorbent article according to any one of claims 1 to 4, wherein
a third air curtain forming member is positioned at a location on an upstream side of the head in a direction of travel of the continuous sheet, to oppose the head with a space therebetween, and forms a third air curtain by emitting air toward the one side of the continuous sheet.

6. An adhesive application device related to an absorbent article according to claim 5, wherein
the pair of second air curtain forming members, by being continuously provided to the first air curtain forming member and the third air curtain forming member, are positioned to surround the head from at least four sides being a downstream side and upstream side in the direction of travel and both sides in the width direction,
the first emitting hole of a slit shape that emits the air related to the first air curtain is provided to a face, of the first air curtain forming member, that opposes the one side of the continuous sheet,
the second emitting holes of slit shapes that emit the air relating to the second air curtains are provided to each faces, of the pair of second air curtain forming members, that oppose the one side of the continuous sheet,
the third emitting hole of a slit shape that emits the air relating to the third air curtain is provided to a face, of the third air curtain forming member, that opposes the one side of the continuous sheet, and
the second emitting holes are connected to the first emitting hole and the third emitting hole.

7. A method of applying adhesive on one side of a continuous sheet that travels, by acting air discharged from an air discharge hole of a head on the adhesive discharged from an adhesive discharge hole of the head, the adhesive application method related to an absorbent article comprising:
forming a first air curtain by positioning a first air curtain forming member at a location on a downstream side of the head in a direction of travel of the continuous sheet, to oppose the head with a space therebetween, and emitting from the first air curtain forming member air toward the one side of the continuous sheet; and
forming second air curtains by positioning second air curtain forming members at locations at outer sides from head in a width direction of the continuous sheet, to respectively oppose the head with spaces therebetween, and emitting from the second air curtain forming members air toward the one side of the continuous sheet.
